# FASCICULE DE BREVET EUROPEEN

(11) **EP 0 622 349 B1**
(45) Date de publication et mention de la délivrance du brevet: **19.02.1997**
(21) Numéro de dépôt: 94400802.8
(22) Date de dépôt: 13.04.1994
(51) Int. Cl.: C07C 17/26, C07C 19/08

(54) **Synthèse d'iodures de perfluoroalkyle**
Synthese von Perfluoralkyljodiden
Synthesis of perfluoroalkyliodides

(30) Priorité: 27.04.1993 FR 9304939
(43) Date de publication de la demande: 02.11.1994
(73) Titulaire: ELF ATOCHEM S.A., 92800 Puteaux (FR)
(72) Inventeur: Bertocchio, René, F-69390 Vourles Par Vernaison (FR); Lambert, Patrick, F-64000 Pau (FR); Lacote, Georges, F-71170 Chauffailles (FR)
(74) Mandataire: Leboulenger, Jean

(56) Documents cités:
- FR-A- 1 415 498
- US-A- 3 404 189

## Description

La présente invention concerne le domaine des hydrocarbures aliphatiques perhalogénés et a plus particulièrement pour objet la préparation des iodures de perfluoroalkyle Rfl, Rf désignant un radical perfluoroalkyle linéaire contenant de 6 à 12 atomes de carbone ou ramifié contenant de 7 à 13 atomes de carbone.

Ces composés sont utilisés comme intermédiaires de synthèse pour de nombreuses applications concernant d'une manière générale le domaine des substances tensio-actives fluorées et plus particulièrement les bases pour formulations extinctrices, les apprêts hydrophobes et oléophobes pour le traitement des textiles ou du papier, et plus récemment les applications à caractère médical (agents de contraste ou transporteurs d'oxygène).

Les iodures de perfluoroalkyle linéaires sont habituellement obtenus par télomérisation du tétrafluoroéthylène par l'iodure de pentafluoroéthyle C₂F₅l, lui-même préparé par action de l'iode et du pentafluorure d'iode sur le tétrafluoroéthylène en présence d'un catalyseur. Ces deux réactions peuvent être couplées comme décrit dans le brevet FR 1 385 682, mais dans la plupart des cas on prépare d'abord C₂F₅l et on l'utilise ensuite dans la télomérisation. L'accès aux iodures de perfluoroalkyle ramifiés se fait à partir d'un iodure de perfluoroalkyle secondaire tel que l'iodure d'heptafluoroisopropyle CF₃CFlCF₃.

La réaction de télomérisation peut être réalisée selon au moins trois méthodes se distinguant essentiellement par le mode d'activation qui peut être :
- soit radicalaire à l'aide de divers amorceurs péroxydiques comme dans les procédés faisant l'objet des brevets FR 2 035 913, FR 2 325 665 et US 3 226 449,
- soit catalytique par l'intervention d'un système rédox comme dans les procédés selon les brevets FR 2 028 781 et FR 2 098 335,
- soit enfin thermique comme dans les procédés faisant l'objet des brevets FR 1 415 198 et US 3 404 189.

Dans tous ces procédés, il est obtenu une distribution plus ou moins large des différentes longueurs de chaîne et, même dans les procédés à initiation catalytique réputés plus sélectifs, il est difficile de parvenir à une distribution relativement étroite pour un télomère de rang j, j allant de 2 à 5 et désignant le nombre de molécules de tétrafluoroéthylène télomérisées par l'iodure de pentafluoroéthyle ou d'heptafluoroisopropyle.

Il est bien connu que cette télomérisation présente la particularité de conduire à des produits pouvant à leur tour fonctionner comme télogène et contribuer ainsi à l'allongement des chaînes, fonction à peu près exclusivement assurée par les réactions de propagation dans la plupart des télomérisations. Dans tout ce qui suit, tout iodure de perfluoroalkyle pourra être considéré à la fois comme un télomère ou un télogène de rang i, C₂F₅l ou CF₃CFlCF₃ étant par définition seulement le télogène de rang 0.

Dans un grand nombre d'applications, il est possible d'utiliser l'ensemble des télomères ou tout au moins des coupes de rang i à j, j étant un nombre entier égal ou supérieur à i+1. Par contre certaines applications nécessitent la mise en oeuvre de produits présentant une longueur de chaîne perfluorée bien définie.

A partir d'un télogène de rang i, il est relativement aisé d'obtenir le télomère de rang i+1 avec une bonne sélectivité en diminuant le taux de conversion par tout moyen adapté (température, temps de contact, rapport molaire télogène/C₂F₄). Pour un télomère de rang j tel que j > i+1, le problème est plus difficile et l'optimisation ne peut guère se faire qu'en recyclant totalement ou partiellement les télomères de rang i+1 à j-1 avec le risque d'accroître la proportion de télomères de rang supérieur à j, c'est-à-dire la fraction de produits lourds indésirables.

La demande de brevet EP 0 433 988 décrit un moyen plus élaboré pour améliorer la productivité et orienter la réaction vers la formation de certains composés présentant notamment des longueurs de chaînes carbonées bien définies. Pour atteindre cet objectif, une partie du mélange réactionnel liquide est prélevée dans la deuxième moitié d'un réacteur tubulaire et réinjectée, par l'intermédiaire d'une deuxième boucle, dans la première moitié du tube de telle sorte que l'espace réactionnel compris entre les deux points ainsi définis représente les 20 à 90 % du volume réactionnel total. En sortie de réacteur le mélange réactionnel est fractionné ; les produits lourds intéressants sont soutirés de l'installation et les produits légers et les réactifs non consommés sont renvoyés en tête du réacteur où ils rejoignent l'alimentation en réactifs frais. En fait, si l'on prend en compte la somme des produits recyclés par la première et par la deuxième boucle, un tel procédé entraîne un volume de recyclat très important par rapport à la productivité de l'installation, la masse de produits recyclés étant 80 à 200 fois plus importante que la production effective selon les exemples cités. Ce procédé ne réduit pas par ailleurs la production de télomères lourds puisque les rapports C₈F₁₇l/C₁₀F₂₁l + télomères de rangs supérieurs sont respectivement égaux à 2,1 et 1,45 pour des taux de recyclage globaux de 200 et 80. L'introduction d'une deuxième boucle de recyclage augmente la productivité du système d'environ 20 à 30 % mais génère une trop forte proportion de produits lourds peu ou non valorisables et les gains en sélectivité sur le C₈F₁₇l ne peuvent être obtenus qu'au prix d'un dimensionnement disproportionné des circuits de recyclage.

Le but de la présente invention est de parvenir à partir d'un télogène de rang i à la production optimale en télomère de rang j avec j > i+1 sans accroître d'une part la production de produits plus lourds et sans faire chuter la productivité d'une manière exagérée.

Il a été découvert à ce propos que, si l'on opère en phase gazeuse dans les conditions de la télomérisation thermique et si celle-ci est réalisée dans un réacteur tubulaire, le recyclage des télomères de rang i+1 à j-1 dans la zone du réacteur comprise entre le vingtième et les trois-quarts de sa longueur conduit à une amélioration simultanée de la productivité et de la sélectivité en télomère de rang j par opposition au cas où la totalité du recyclat est réintroduite avec les réactifs frais en tête du tube.

L'invention a donc pour objet un procédé continu de préparation des iodures de perfluoroalkyle Rfl par télomérisation thermique en phase gazeuse du tétrafluoroéthylène par l'iodure de pentafluoroéthyle ou d'heptafluoroisopropyle (i=0) ou par un télomère inférieur de rang i allant de 1 à 3, en réacteur tubulaire, caractérisé en ce que les télomères de rang i+1 à j-1, j désignant le rang du télomère désiré, sont recyclés en au moins un point du réacteur situé entre le vingtième et les trois quarts de la longueur du tube, de préférence entre le cinquième et les deux cinquièmes, ces distances étant comptées à partir de l'entrée du tube.

Ce procédé est avantageusement réalisé dans un réacteur tubulaire en acier inoxydable ou en nickel de forme quelconque et de rapport longueur sur diamètre intérieur compris entre 50 et 5000 chauffé d'une manière homogène sur toute sa longueur par un dispositif approprié à une température comprise entre 300 et 365°C. Le tube comporte, à diverses distances de son extrémité d'entrée, un certain nombre de piquages permettant le recyclage des télomères de rang i+1 à j-1.

L'ensemble des télomères de rang i+1 à j-1 peut être recyclé en un même point du réacteur tubulaire. On peut également procéder à un recyclage étagé dans lequel les téloméres de rang i+1 à j-1 sont recyclés en différents points du réacteur en fonction de leur rang, les télomères les plus légers étant avantageusement injectés dans une zone amont du réacteur située entre le vingtième et le tiers de sa longueur et les plus lourds dans une zone aval située entre le cinquième et la moitié de la longueur du tube.

Les réactifs de départ, à savoir le taxogéne (C₂F₄) et le télogène de rang i, sont introduits en tête du réacteur par tout moyen approprié, par exemple à l'aide d'une pompe doseuse pour le télogène et d'un dispositif de régulation de débit gazeux pour C₂F₄.

Au lieu d'introduire la totalité du tétrafluoroéthylène en tête du réacteur, on peut en introduire une partie (25 à 70 %, avantageusement 40 à 60 %) en au moins un point du tube situé entre les deux cinquièmes et les trois quarts de la longueur du tube. Il a en effet été montré dans la demande de brevet EP 0552076 qu'une telle alimentation étagée permet de réduire la proportion de télomères lourds.

Les produits sortant du réacteur de télomérisation sont introduits dans une colonne de trempe pour séparer et renvoyer en tête de réacteur le C₂F₄ et le télogène de départ non transformés. Les télomères sont ensuite fractionnés dans une première colonne pour isoler les télomères de rang i+1 à j-1 et les renvoyer dans le réacteur au point le mieux adapté à la production sélective du télomère de rang j, puis dans une deuxième colonne pour séparer le télomère de rang j des produits plus lourds.

Conformément à un mode particulier de mise en oeuvre du procédé selon l'invention, l'alimentation du réacteur en réactifs frais et/ou recyclés est avantageusement réglée de façon à ce qu'en régime stationnaire les quantités de télomère de rang i+1 entrant et sortant du réacteur soient sensiblement identiques.

Le rapport molaire des réactifs frais (C₂F₄ et télogène de départ) alimentés au réacteur dépend du rang j du télomère désiré et de la sélectivité recherchée. Le rapport molaire télogène frais/C₂F₄ frais peut aller de 0,1 à 0,6 et est de préférence compris entre 0,2 et 0,5.

Pour atteindre un état de régime stationnaire pour les télomères de rang k intermédiaire (i+1 ≤ k ≤ j-1) et pour améliorer simultanément sélectivité et productivité en télomère de rang j, les rapports molaires Tk = Télogène de rang k/télogène de rang i, seront utilement compris entre 0,2 et 2, préférentiellement entre 0,5 et 1,4. Ces rapports sont avantageusement pris sensiblement égaux entre eux sans que cela constitue une limitation au procédé.

En pratique, le système est amorcé avec un pied de télogène de rang i à j-1 dans les proportions définies ci-dessus et une alimentation en réactifs frais (télogène de rang i et C₂F₄) définie par le rang du télomère j, la sélectivité et la productivité cherchée.

La réaction de télomérisation peut être conduite dans un domaine de température allant de 300 à 360°C, mais elle est avantageusement effectuée à une température comprise entre environ 325 et 355°C.

Industriellement, on peut travailler à la pression atmosphérique ou à une pression supérieure à la pression atmosphérique, pourvu que le système réactionnel reste à l'état gazeux.

Les exemples suivants, donnés à titre non limitatif, illustrent l'invention dans le cas de la fabrication du iodure de perfluorooctyle C₈F₁₇l (j=3) à partir du iodure de perfluoroéthyle (i=0). Les pourcentages indiqués sont exprimés en poids.

Le C₂F₅l utilisé est pur à 99,87 %, les principales impuretés étant du C₄F₉l (0,06 %) et C₂F₄ (0,055 %).

Le C₄F₉l utilisé est pur à 98,6 %, les principales impuretés étant C₂F₅l (0,1 %), C₆F₁₃l (0,2%) et des perfluoroalcanes RfRf, en particulier C₈F₁₈ et C₁₀F₂₂ (0,3 %).

Le C₆F₁₃l utilisé est pur à 99,55 %, les principales impuretés étant C₈F₁₇l (0,1 %) et des perfluoroalcanes Rf-Rf (0,25 %).

Les bilans entrée et sortie des différents composants du mélange réactionnel permettent de calculer:
- la composition de l'alimentation en réactifs frais (C₂F₄ et C₂F₅l),
- la composition de la production en télomères,
- la productivité en télomère de rang j exprimée en grammes/heure/litre de réacteur,
- la sélectivité exprimée par le rapport des masses de télomère de rang j et des télomères de rangs supérieurs à j.

### EXEMPLE A (Comparatif)

On utilise un réacteur constitué d'un tube de nickel de 20 m de long et 4,3 mm de diamètre interne, enroulé en spirale autour d'un mandrin chauffant permettant de maintenir la température du tube à 350 ± 5°C sur l'ensemble de sa longueur.

On introduit en tête du réacteur et par l'intermédiaire de trois pompes doseuses respectivement 229,8 g/h de C₂F₅l, 121,8 g/h de C₄F₉l et 125,34 g/h de C₆F₁₃l, ainsi que 19,46 g/h de C₂F₄ gazeux par un dispositif de régulation massique.

Le mélange réactionnel sortant du réacteur est condensé à travers un réfrigérant à eau et séparé en une phase gazeuse et une phase liquide dont les compositions sont déterminées par chromatographie en phase gazeuse.

Les bilans massiques entrée et sortie sont rassemblés dans le tableau suivant.

| **CONSTITUANT** | **ENTREE (g/h/l)** | **SORTIE (g/h/l)** |
|---|---|---|
| C₂F₄ | 67,55 | 26,2 |
| C₂F₅l | 791,9 | 756,2 |
| C₄F₉l | 414,8 | 420,5 |
| C₆F₁₃l | 431,1 | 440,9 |
| C₈F₁₇l | 0,45 | 53,45 |
| C₁₀F₂₁l | - | 6,9 |
| ≥ C₁₂F₂₅l | - | 0,8 |
| RfRf | 3,9 | 7,5 |

Dans ces conditions expérimentales qui correspondent à une alimentation du réacteur avec 41,3 g/h/l de C₂F₄ frais et 35,7 g/h/l de C₂F₅l frais, la production de télomères a la répartition suivante :

| | |
|---|---|
| C₄F₉l | 7,5 % |
| C₆F₁₃l | 12,9 % |
| C₈F₁₇l | 69,6 % |
| C₁₀F₂₁l | 9,0 % |
| ≥ C₁₂F₂₅l | 1,0 % |

La sélectivité s'élève à 6,96 pour une productivité en C₈F₁₇l de 53 g/heure et par litre de réacteur.

### EXEMPLE B(Comparatif)

On utilise un réacteur constitué d'un tube en acier inoxydable de 20 m de long et 4 mm de diamètre interne, enroulé en spirale autour d'un mandrin chauffant permettant de maintenir la température du tube à 350 ± 5°C sur l'ensemble de sa longueur.

On introduit en tête du réacteur et par les mêmes dispositifs que précédemment, 39,2 g/h de C₂F₄, 97,8 g/h de C₂F₅l, 27,6 g/h de C₄F₉l et 41,4 g/h de C₆F₁₃l. Dans ces conditions la composition du mélange réactionnel sortant du tube à 344°C est telle que la masse de C₄F₉l recueillie est égale à la masse de C₄F₉l introduite.

Les bilans massique entrée et sortie sont rassemblés dans le tableau suivant :

| **CONSTITUANT** | **ENTREE (g/h/l)** | **SORTIE (g/h/l)** |
|---|---|---|
| C₂F₄ | 157,1 | 65,2 |
| C₂F₅l | 391,1 | 324,7 |
| C₄F₉l | 108,4 | 108,9 |
| C₆F₁₃l | 163,8 | 188,3 |
| C₈F₁₇l | 0,1 | 72,5 |
| C₁₀F₂₁l | 0 | 35,5 |
| ≥ C₁₂F₂₅l | 0 | 25,2 |
| RfRf | 1 | 1,2 |

Ces conditions de fonctionnement correspondent à une alimentation du réacteur avec 91,9 g/h/l de C₂F₄ frais et 66,4 g/h/l de C₂F₅l frais. La production de télomères a la répartition suivante :

| | |
|---|---|
| C₄F₉l | 0,3 % |
| C₆F₁₃l | 15,5 % |
| C₈F₁₇l | 45,8 % |
| C₁₀F₂₁l | 22,5 % |
| ≥ C₁₂F₂₅l | 15,9 % |

La productivité en C₈F₁₇l atteint 72,4 g/h/l, mais la sélectivité tombe à 1,2.

### EXEMPLE C (Comparatif)

On utilise le réacteur de l'exemple A qu'on alimente en tête par le mélange de 30,5 g/h de C₂F₄, 354,5 g/h de C₂F₅l, 184,4 g/h de C₄F₉l et 193,8 g/h de C₆F₁₃l. A 350°C on obtient sensiblement un équilibre au niveau du C₄F₉l et du C₆F₁₃l. Les bilans massiques entrée et sortie sont rassemblés dans le tableau suivant :

| **CONSTITUANT** | **ENTREE (g/h/l)** | **SORTIE (g/h/l)** |
|---|---|---|
| C₂F₄ | 106 | 51,7 |
| C₂F₅l | 1221,6 | 1179,6 |
| C₄F₉l | 627,7 | 645,8 |
| C₆F₁₃l | 667,8 | 673,9 |
| C₈F₁₇l | 0,7 | 60,7 |
| C₁₀F₂₁l | 0 | 8,4 |
| ≥ C₁₂F₂₅l | 0 | 1,2 |
| RfRf | 6 | 8,6 |

Ces conditions de fonctionnement correspondent à une alimentation du réacteur avec 54,3 g/h/l de C₂F₄ frais et 42 g/h/l de C₂F₅l frais. La production de télomères a la répartition suivante :

| | |
|---|---|
| C₄F₉l | 19,3 % |
| C₆F₁₃l | 6,5 % |
| C₈F₁₇l | 64 % |
| C₁₀F₂₁l | 9,0 % |
| ≥ C₁₂F₂₅l | 1,3 % |

La productivité en C₈F₁₇l atteint 60 g/h/l, et la sélectivité s'élève à 6,25.

### EXEMPLE 1

On utilise le même réacteur qu'à l'exemple A, mais on introduit les télomères de rangs 1 et 2 (C₄F₉l et C₆F₁₃l) par un piquage situé au tiers de la longueur du tube.

A 350°C, le régime sensiblement stationnaire au niveau de C₄F₉l est atteint en alimentant le réacteur de la façon suivante :
- en tête du réacteur: 23,5 g/h de C₂F₄
   et 130,1 g/h de C₂F₅l
- au 1/3 du réacteur: 231,4 g/h de C₄F₉l
   et 265,16 g/h de C₆F₁₃l.

Les bilans massiques entrée et sortie sont rassemblés dans le tableau suivant :

| **CONSTITUANT** | **ENTREE (g/h/l)** | **SORTIE (g/h/l)** |
|---|---|---|
| C₂F₄ | 81,5 | 26,1 |
| C₂F₅l | 448,8 | 399,2 |
| C₄F₉l | 787,1 | 797,6 |
| C₆F₁₃l | 911,8 | 925,5 |
| C₈F₁₇l | 0,9 | 68,1 |
| C₁₀F₂₁l | - | 7,4 |
| ≥ C₁₂F₂₅l | - | 1,7 |
| RfRf | 7,7 | 15,9 |

Dans ces conditions qui correspondent à une alimentation du réacteur avec 55,4 g/h/l de C₂F₄ frais et 49,6 g/h/l de C₂F₅l frais, la production de télomères a la répartition suivante :

| | |
|---|---|
| C₄F₉l | 10,4 % |
| C₆F₁₃l | 13,6 % |
| C₈F₁₇l | 66,9 % |
| C₁₀F₂₁l | 7,4 % |
| ≥ C₁₂F₂₅l | 1,7 % |

La sélectivité s'élève à 7,35 pour une productivité en C₈F₁₇l de 67,2 g/h/l. Par rapport à l'exemple comparatif A, les gains en sélectivité et en productivité sont respectivement de 5,6 % et de 26,8 %.

Par rapport à l'exemple C réalisé à un débit de réactifs frais sensiblement comparables, les gains en sélectivité et en productivité sont respectivement égaux à 17,5 % et 12 %.

### EXEMPLE 2

On répète l'exemple 1, mais avec un piquage situé au quart de la longueur du tube.

A 350°C, on atteint un régime stationnaire au niveau du C₄F₉l en alimentant le réacteur de la façon suivante :
- en tête du réacteur : 23,7 g/h de C₂F₄
   et 119,6 g/h de C₂F₅l
- au 1/4 du réacteur: 229,4 g/h de C₄F₉l
   et 294,6 g/h de C₆F₁₃l

Les bilans massiques entrée et sortie sont rassemblés dans le tableau suivant :

| **CONSTITUANT** | **ENTREE (g/h/l)** | **SORTIE (g/h/l)** |
|---|---|---|
| C₂F₄ | 81,7 | 28,2 |
| C₂F₅l | 412,8 | 355,2 |
| C₄F₉l | 780,3 | 780,0 |
| C₆F₁₃l | 1013,1 | 1033,2 |
| C₈F₁₇l | 1,0 | 78,7 |
| C₁₀F₂₁l | - | 8,7 |
| ≥ C₁₂F₂₅l | - | 1,4 |
| RfRf | 8 | 13,3 |

Dans ces conditions qui correspondent à une alimentation du réacteur avec 53,5 g/h/l de C₂F₄ frais et 57,6 g/h/l de C₂F₅l frais, la production de télomères a la répartition suivante :

| | |
|---|---|
| C₆F₁₃l | 18,6 % |
| C₈F₁₇l | 72 % |
| C₁₀F₂₁l | 8 % |
| ≥ C₁₂F₂₅l | 1,3 % |

La sélectivité s'élève à 7,75 pour une productivité en C₈F₁₇l de 77,7 g/h/l.

Par rapport à l'exemple comparatif A, les gains en sélectivité et en productivité sont respectivement de 11,4 % et de 46,6 %.

### EXEMPLE 3

On utilise le même réacteur qu'à l'exemple B avec introduction des télomères de rangs 1 et 2 (C₄F₉l et C₆F₁₃l) par un piquage situé au tiers de la longueur du tube.

A 340°C on parvient à un régime sensiblement stationnaire au niveau du C₄F₉l en alimentant le réacteur de la façon suivante :
- en tête du réacteur : 25,6 g/h de C₂F₄
   et 94,4 g/h de C₂F₅l
- au 1/3 du réacteur : 74,0 g/h de C₄F₉l
   et 112,8 g/h de C₆F₁₃l

Les bilans massiques entrée et sortie sont rassemblés dans le tableau suivant :

| **CONSTITUANT** | **ENTREE (g/h/l)** | **SORTIE (g/h/l)** |
|---|---|---|
| C₂F₄ | 102,4 | 28,5 |
| C₂F₅l | 377,4 | 294 |
| C₄F₉l | 292,1 | 320,3 |
| C₆F₁₃l | 449,8 | 467,7 |
| C₈F₁₇l | 0,4 | 81,9 |
| C₁₀F₂₁l | 0 | 18,6 |
| ≥ C₁₂F₂₅l | 0 | 8,9 |
| RfRf | 2,8 | 4,3 |

Dans ces conditions qui correspondent à une alimentation du réacteur avec 73,9 g/h/l de C₂F₄ frais et 83,4 g/h/l de C₂F₅l frais, la production de télomères a la répartition suivante :

| | |
|---|---|
| C₄F₉l | 18,2 % |
| C₆F₁₃l | 11,6 % |
| C₈F₁₇l | 52,5 % |
| C₁₀F₂₁l | 12,0 % |
| ≥ C₁₂F₂₅l | 5,7 % |

La productivité en C₈F₁₇l atteint 81,5 g/h/l et la sélectivité s'élève à 2,96.

Par rapport à l'exemple B et pour un débit d'alimentation en réactifs frais sensiblement identique, le recyclage étagé augmente la productivité en C₈F₁₇l de 12,5 % alors que la sélectivité est multipliée par 2,5.

## Revendications

1. Procédé continu de préparation des iodures de perfluoroalkyle Rfl, Rf désignant un radical perfluoroalkyle linéaire contenant de 6 à 12 atomes de carbone ou ramifié contenant de 7 à 13 atomes de carbone, par télomérisation thermique en phase gazeuse du tétrafluoroéthylène par l'iodure de pentafluoroéthyle ou d'heptafluoroisopropyle (i=0) ou par un télomère inférieur (i=1 à 3), dans un réacteur tubulaire, caractérisé en ce que les télomères de rang i+1 à j-1, j désignant le rang du télomère désiré, sont recyclés en au moins un point du réacteur situé entre le vingtième et les trois quarts de la longueur du tube.

2. Procédé selon la revendication 1, dans lequel le recyclage des télomères de rang i+1 à j-1 est effectué en au moins un point du tube situé entre le cinquième et les deux cinquièmes de sa longueur.

3. Procédé selon la revendication 1 ou 2, dans lequel les télomères de rang i+1 à j-1 sont recyclés en différents points du réacteur, les plus légers dans une zone amont du réacteur et les plus lourds dans une zone aval.

4. Procédé selon la revendication 3, dans lequel la zone amont est située entre le vingtième et le tiers de la longueur du tube et la zone aval entre le cinquième et la moitié de la longueur du tube.

5. Procédé selon l'une des revendications 1 à 4, dans lequel le rapport longueur/diamètre intérieur du réacteur tubulaire est compris entre 50 et 5000.

6. Procédé selon l'une des revendications 1 à 5, dans lequel la télomérisation est effectuée à une température allant de 300 à 360°C, de préférence entre environ 325 et 355°C.

7. Procédé selon l'une des revendications 1 à 6, dans lequel le rapport molaire télogène frais/C₂F₄ frais est compris entre 0,1 et 0,6, de préférence entre 0,2 et 0,5.

8. Procédé selon l'une des revendications 1 à 7, dans lequel une partie du tétrafluoroéthylène est injectée en au moins un point du réacteur situé entre les deux cinquièmes et les trois quarts de la longueur du tube, le reste étant introduit en tête du réacteur en même temps que le télogène de départ.

9. Procédé selon l'une des revendications 1 à 8, dans lequel l'alimentation du réacteur en réactifs frais et/ou recyclés est réglée de façon à ce qu'en régime stationnaire les quantités de télomère de rang i+1 entrant et sortant du réacteur soient sensiblement identiques.

10. Application du procédé selon l'une des revendications 1 à 9 à la fabrication du iodure de perfluorooctyle C₈F₁₇l.

## Claims

1. Continuous process for the preparation of perfluoroalkyl iodides RfI, Rf denoting a perfluoroalkyl radical which is linear and contains from 6 to 12 carbon atoms or is branched and contains from 7 to 13 carbon atoms, by thermal telomerization, in the gas phase in a tubular reactor, of tetrafluoroethylene with pentafluoroethyl iodide or heptafluoroisopropyl iodide (i=0) or with a lower telomer (i=1 to 3), characterized in that the telomers of extent i+1 to j-1, j denoting the extent of the desired telomer, are recycled to at least one point of the reactor which is situated between one-twentieth and three-quarters the length of the tube.

2. Process according to Claim 1, in which the telomers of extent i+1 to j-1 are recycled to at least one point of the tube which is situated between one-fifth and two-fifths of its length.

3. Process according to Claim 1 or 2, in which the telomers of extent i+1 to j-1 are recycled to different points of the reactor, the lighter telomers in an upstream zone of the reactor and the heavier ones in a downstream zone.

4. Process according to Claim 3, in which the upstream zone is situated between one-twentieth and one-third of the length of the tube and the downstream zone is situated between one-fifth and half the length of the tube.

5. Process according to one of Claims 1 to 4, in which the ratio of length to internal diameter of the tubular reactor is between 50 and 5,000.

6. Process according to one of Claims 1 to 5, in which telomerization is carried out at a temperature ranging from 300 to 360°C, preferably between about 325 and 355°C.

7. Process according to one of Claims 1 to 6, in which the molar ratio of fresh telogen to fresh C₂F₄ is between 0.1 and 0.6, preferably between 0.2 and 0.5.

8. Process according to one of Claims 1 to 7, in which one portion of the tetrafluoroethylene is injected at at least one point of the reactor which is situated between two-fifths and three-quarters the length of the tube, the remainder being introduced at the head of the reactor at the same time as the starting telogen.

9. Process according to one of Claims 1 to 8, in which the feeding of the reactor with fresh and/or recycled reactants is regulated such that, in steady-state operation, the quantities of telomer of extent i+1 entering and leaving the reactor are substantially identical.

10. Application of the process according to one of Claims 1 to 9 to the production of perfluorooctyl iodide C₈F₁₇I.

## Patentansprüche

1. Kontinuierliches Verfahren zur Herstellung von Perfluoralkyliodiden RfI, wobei Rf einen linearen Perfluoralkylrest mit 6 bis 12 Kohlenstoffatomen oder einen verzweigten Perfluoralkylrest mit 7 bis 13 Kohlenstoffatomen bezeichnet, durch thermische Gasphasentelomerisation von Tetrafluorethylen mit Pentafluorethyliodid oder Heptafluorisopropyliodid (i = 0) oder mit einem kleineren Telomeren (i=1 bis 3) in einem Rohrreaktor, dadurch gekennzeichnet, daß die Telomeren mit Größe i+1 bis j-1, wobei j die Größe des gewünschten Telomeren bezeichnet, an mindestens einer Stelle des Reaktors wiedereingesetzt werden, die sich zwischen ¹/₂₀ und ¾ der Länge des Rohres befindet.

2. Verfahren nach Anspruch 1, bei dem der Wiedereinsatz der Telomeren mit einer Größe von i+1 bis j-1 an mindestens einer Stelle des Rohres durchgeführt wird, die sich zwischen ¹/₅ und ²/₅ seiner Länge befindet.

3. Verfahren nach Anspruch 1 oder 2, bei dem die Telomeren mit einer Größe von i+1 bis j-1 an verschiedenen Stellen des Reaktors wiedereingesetzt werden, die leichteren in einer oberen Zone des Reaktors und die schwereren in einer unteren Zone des Reaktors.

4. Verfahren nach Anspruch 3, bei dem die obere Zone zwischen ¹/₂₀ und ¹/₃ der Länge des Rohres und die untere Zone zwischen ¹/₅ und der Hälfte der Länge des Rohres gelegen ist.

5. Verfahren nach einem der Ansprüche 1 bis 4, bei dem das Verhältnis Länge/innerer Durchmesser des Rohrreaktors zwischen 50 und 5000 beträgt.

6. Verfahren nach einem der Ansprüche 1 bis 5, bei dem die Telomerisation bei einer Temperatur von 300 bis 360 °C, vorzugsweise zwischen etwa 325 und 355 °C, durchgeführt wird.

7. Verfahren nach einem der Ansprüche 1 bis 6, bei dem das Molverhältnis frisches Telogen/frisches C₂F₄ zwischen 0,1 und 0,6, vorzugsweise zwischen 0,2 und 0,5, liegt.

8. Verfahren nach einem der Ansprüche 1 bis 7, bei dem ein Teil des Tetrafluorethylens an mindestens einer Stelle des Reaktors eingespritzt wird, die sich zwischen ²/₅ und ¾ der Länge des Rohres befindet, wobei der Rest gleichzeitig mit dem Ausgangstelogen am Kopf des Reaktors eingebracht wird.

9. Verfahren nach einem der Ansprüche 1 bis 8, bei dem die Zufuhr an frischen und/oder wiedereingesetzten Reagenzien in dem Reaktor so eingestellt wird, daß im stationären Zustand die Mengen an Telomer mit einer Größe von i+1, die in den Reaktor eintreten oder aus den Reaktor kommen, etwa identisch sind.

10. Anwendung des Verfahrens nach einem der Ansprüche 1 bis 9 zur Herstellung von Perfluoroctyliodid C₈F₁₇I.
